(19)

Europäisches
Patentamt
European
Patent Office
Office européen
des brevets

(11)  **EP 3 843 101 A1**

(12)  **EUROPEAN PATENT APPLICATION**
published in accordance with Art. 153(4) EPC

(43) Date of publication:
**30.06.2021  Bulletin 2021/26**

(21) Application number: **20744928.1**

(22) Date of filing: **10.01.2020**

(51) Int Cl.:
*G16B 20/20* (2019.01)    *G16B 30/00* (2019.01)
*G16B 40/00* (2019.01)    *G16B 50/00* (2019.01)

(86) International application number:
**PCT/KR2020/000436**

(87) International publication number:
**WO 2020/153636 (30.07.2020 Gazette 2020/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority:  **25.01.2019  KR 20190009806
31.12.2019  KR 20190179474**

(71) Applicant: **Genealogy Inc.
Seoul 08789 (KR)**

(72) Inventors:
• **HAN, Buhm
Seoul 01687 (KR)**
• **COOK, Seung Ho
Seoul 07376 (KR)**

(74) Representative: **Nederlandsch Octrooibureau
P.O. Box 29720
2502 LS The Hague (NL)**

(54)  **METHOD FOR PREDICTING GENOTYPE BY USING SNP DATA**

(57)    A method for predicting a genotype using SNP data is disclosed. An embodiment includes steps of receiving SNP data to be analyzed and reference data, of updating the reference data by inserting a marker corresponding to a genotype of the SNP data corresponding to each of a plurality of predetermined regions included in corresponding SNP data, for each SNP data included in the reference data, and of predicting a genotype of the SNP data to be analyzed based on the SNP data to be analyzed and the updated reference data.

FIG. 1

**Description**

TECHNICAL FIELD

The following example embodiments relate to a method of predicting a genotype using single nucleotide polymorphism (SNP) data, and more particularly, to a method of imputing genotypes.

BACKGROUND ART

Deoxyribonucleic acid (DNA) present on chromosomes in cells of organisms including humans is a genetic material passed to offspring during reproduction and propagation. In humans, DNA inherited from each parent is present in chromosome pairs. A part of a DNA sequence involved in gene expression is called a gene, and a structure and a function of an organism are formed by synthesizing proteins by gene expression. Different genotypes of organisms are determined due to a difference in a DNA sequence of a gene. DNA sequences of individuals belonging to the same species contain single nucleotides that differ from individual to individual. The genetic diversity caused by a difference between single nucleotides in DNA sequences is called a single nucleotide polymorphism (SNP). By analyzing single nucleotides that differ from individual to individual, it is possible to predict a genotype of a specific individual.

DISCLOSURE OF INVENTION

TECHNICAL SUBJECT

Example embodiments may provide a technology of predicting a genotype of single nucleotide polymorphism (SNP) data to be analyzed, by inserting a marker corresponding to each of genotypes of a gene to be analyzed into a plurality of regions of SNP data included in reference data.

Also, example embodiments may provide a technology of predicting a genotype of SNP data to be analyzed, based on the SNP data to be analyzed, reference data, and a genetic distance between SNP data with determined genotypes.

TECHNICAL SOLUTION

According to an aspect, there is provided a method of predicting a genotype using SNP data which includes: acquiring SNP data to be analyzed; acquiring reference data including a plurality of pieces of SNP data with determined genotypes; updating the reference data by inserting a marker corresponding to a genotype of corresponding SNP data into each of a plurality of predetermined regions included in the corresponding SNP data, for each of the plurality of pieces of SNP data included in the reference data; and predicting a genotype of the SNP data to be analyzed, based on the SNP data to be analyzed and the updated reference data.

The updating of the reference data may include inserting a binary marker corresponding to the genotype of the corresponding SNP data into a plurality of exons included in the corresponding SNP data, for each of the plurality of pieces of SNP data included in the reference data.

The predicting of the genotype of the SNP data to be analyzed may include: calculating probabilities that the SNP data to be analyzed corresponds to the genotypes of the plurality of pieces of SNP data for each region, by inputting the SNP data to be analyzed and the updated reference data to a prediction model; and predicting the genotype of the SNP data to be analyzed, based on the probabilities.

The predicting of the genotype of the SNP data to be analyzed may include: setting a plurality of parameters indicating lengths of nucleic acid sequences for analyzing the SNP data to be analyzed, based on the plurality of pieces of SNP data included in the updated reference data; calculating probabilities that the SNP data to be analyzed corresponds to the genotypes of the plurality of pieces of SNP data for each combination of the regions and the parameters, by inputting the parameters, the SNP data to be analyzed and the updated reference data to a prediction model; and determining the genotype of the SNP data to be analyzed, based on the probabilities.

The predicting of the genotype of the SNP data to be analyzed may include: calculating a genetic distance between a plurality of markers corresponding to the genotypes of the plurality of pieces of SNP data; and predicting the genotype of the SNP data to be analyzed, based on the genetic distance, the SNP data to be analyzed, and the updated reference data.

The calculating of the genetic distance may include: sampling the SNP data to be analyzed and the plurality of pieces of SNP data; calculating a transition probability between states corresponding to the genotypes of the plurality of pieces of SNP data in a hidden Markov model (HMM), based on the sampled data; and acquiring a genetic distance between the states by converting the transition probability between the states.

The method may further include: separating the SNP data to be analyzed into two pieces of haploid data by phasing the SNP data to be analyzed; and obtaining two pieces of diploid data by duplicating each of the two pieces of haploid data and pairing the haploid data and duplicated data of the haploid data.

The predicting of the genotype of the SNP data to be analyzed may include predicting a genotype of corresponding diploid data by inputting the corresponding diploid data and the updated reference data to a prediction model, for each of the two pieces of diploid data.

The separating of the SNP data to be analyzed into the two pieces of haploid data by phasing the SNP data to be analyzed may include separating the SNP data to be analyzed into maternal SNP data and paternal SNP data.

The method may further include determining markers corresponding to the genotypes of the plurality of pieces of SNP data.

The SNP data to be analyzed may include: at least a portion of a DNA sequence of a user to be analyzed; and information of at least a portion of SNPs included in the at least portion of the DNA sequence.

The reference data may include at least one SNP data corresponding to one of a plurality of genotypes defined in a gene from which the SNP data to be analyzed is extracted.

Each of the plurality of pieces of SNP data included in the updated reference data may include: a DNA sequence of a corresponding genotype; information of a SNP included in the DNA sequence; and markers inserted into positions of the regions in the DNA sequence.

The SNP data to be analyzed may include SNP data extracted from a human leukocytic antigen (HLA) gene, and the genotypes may include a plurality of genotypes defined in the HLA gene.

According to another aspect, there is provided a method of predicting a genotype using SNP data which includes: acquiring SNP data to be analyzed; acquiring reference data including a plurality of pieces of SNP data with determined genotypes; sampling the SNP data to be analyzed and the plurality of pieces of SNP data; calculating a transition probability between states corresponding to the genotypes of the plurality of pieces of SNP data in an HMM, based on the sampled data; acquiring a genetic distance between the states by converting the transition probability between the states; and predicting a genotype of the SNP data to be analyzed, based on the genetic distance, the reference data, and the SNP data to be analyzed.

According to another aspect, there is provided an apparatus for predicting a genotype using SNP data which includes: a memory configured to store SNP data to be analyzed, and reference data including a plurality of pieces of SNP data with determined genotypes; and a processor configured to update the reference data by inserting a marker corresponding to a genotype of corresponding SNP data into each of a plurality of predetermined regions included in the corresponding SNP data, for each of the plurality of pieces of SNP data included in the reference data, and to predict a genotype of the SNP data to be analyzed, based on the SNP data to be analyzed and the updated reference data.

To update the reference data, the processor may be configured to insert a binary marker corresponding to the genotype of the corresponding SNP data into a plurality of exons included in the corresponding SNP data, for each of the plurality of pieces of SNP data included in the reference data.

To predict the genotype of the SNP data to be analyzed, the processor may be configured to calculate a genetic distance between a plurality of markers corresponding to the genotypes of the plurality of pieces of SNP data and to predict the genotype of the SNP data to be analyzed, based on the genetic distance, the SNP data to be analyzed, and the updated reference data.

The SNP data to be analyzed may include SNP data extracted from an HLA gene, and the genotypes may include a plurality of genotypes defined in the HLA gene.

BRIEF DESCRIPTION OF DRAWINGS

FIG. 1 is a diagram illustrating an overall flow of a method of predicting a genotype of single nucleotide polymorphism (SNP) data to be analyzed according to an example embodiment.

FIG. 2 is a diagram illustrating a SNP.

FIG. 3 is a diagram illustrating exons and a structure of a chromosome.

FIG. 4 is a diagram illustrating a method of predicting a genotype of SNP data to be analyzed using a prediction model according to an example embodiment.

FIG. 5 is a diagram illustrating a prediction model and a method of predicting a genotype of SNP data to be analyzed based on probabilities for each of a plurality of regions according to an example embodiment.

FIG. 6A is a diagram illustrating a prediction model and a method of predicting a genotype of SNP data to be analyzed by setting a plurality of parameters indicating lengths of nucleic acid sequences for analyzing SNP data according to an example embodiment.

FIG. 6B is a diagram illustrating a hidden Markov model (HMM) according to an example embodiment.

FIG. 7 is a diagram illustrating a method of predicting a genotype of SNP data to be analyzed, based on the SNP data to be analyzed, reference data, and a genetic distance according to an example embodiment.

FIG. 8 is a diagram illustrating a method of acquiring a genetic distance according to an example embodiment.

BEST MODE FOR CARRYING OUT THE INVENTION

The following structural or functional descriptions of example embodiments disclosed in the present disclosure are merely intended for the purpose of describing the example embodiments and the example embodiments may be implemented in various forms. The example embodiments are not meant to be limited, but it is intended that various modifications, equivalents, and alternatives are also covered within the scope of the claims.

Although terms of "first" or "second" are used to explain various components, the components are not limited to the terms. These terms should be used only to distinguish one component from another component. For example, a "first" component may be referred to as a "second" component, or similarly, and the "second" component may be referred to as the "first" component within the scope of the right according to the concept of the present disclosure.

It should be noted that if it is described in the specification that one component is "connected," or "coupled," to another component, a third component may be "connected," and "coupled" between the first and second components, although the first component may be directly connected or coupled to the second component. In addition, it should be noted that if it is described in the specification that one component is "directly connected" or "directly coupled" to another component, a third component may not be present therebetween. Likewise, expressions, for example, "between" and "immediately between" and "adjacent to" and "immediately adjacent to" may also be construed as described in the foregoing.

As used herein, the singular forms "a," "an," and "the" are intended to include the plural forms as well, unless the context clearly indicates otherwise. As used herein, the terms "include," "comprise," and "have" specify the presence of stated features, numbers, operations, elements, components, and/or combinations thereof, but do not preclude the presence or addition of one or more other features, numbers, operations, elements, components, and/or combinations thereof.

Unless otherwise defined, all terms used herein including technical or scientific terms have the same meanings as those generally understood consistent with and after an understanding of the present disclosure. Terms, such as those defined in commonly used dictionaries, should be construed to have meanings matching with contextual meanings in the relevant art and the present disclosure, and are not to be construed as an ideal or excessively formal meaning unless otherwise defined herein.

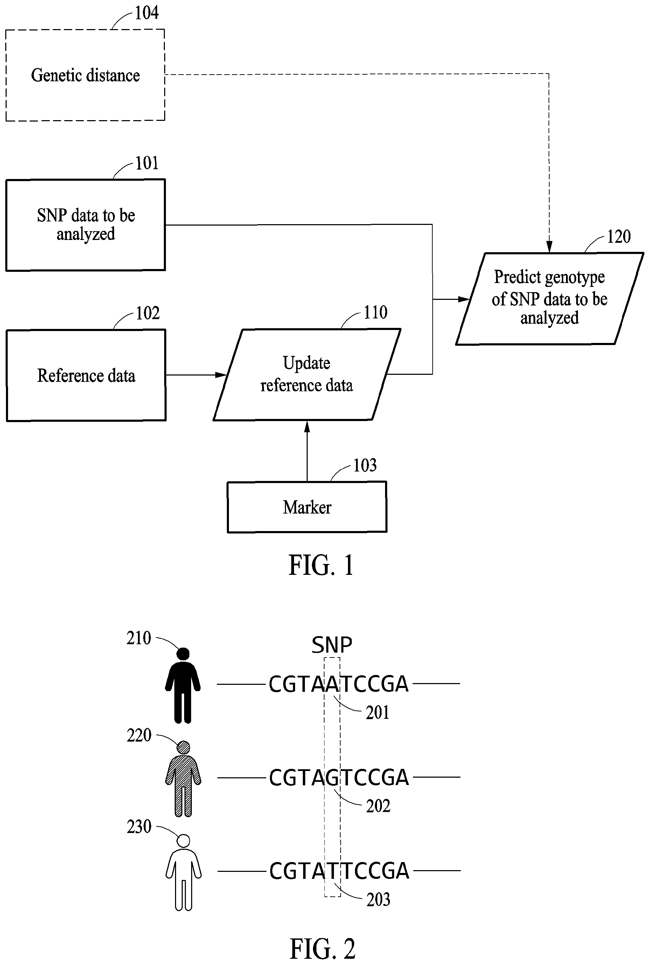

FIG. 1 is a diagram illustrating an overall flow of a method of predicting a genotype of single nucleotide polymorphism (SNP) data to be analyzed according to an example embodiment.

Referring to FIG. 1, a method of predicting a genotype using SNP data may include an operation of acquiring SNP data 101 to be analyzed, an operation of acquiring reference data 102, operation 110 of updating the reference data 102, and operation 120 of predicting a genotype of the SNP data to be analyzed.

A deoxyribonucleic acid (DNA) sequence is a succession of nucleotides that are components of a nucleotide that is a basic unit in DNA. A base of a nucleotide may correspond to one of adenine (A), thymine (T), guanine (G), and cytosine (C). DNA may be present on chromosomes in cells. In humans, each cell contains "23" pairs of chromosomes inherited from each parent. Chromosomes forming one chromosome pair may be called homologous chromosomes, and one of homologous chromosomes of a chromosome pair may have a paternal DNA sequence, and the other homologous chromosome may be a maternal DNA sequence. Genes may have a portion of DNA sequences involved in gene expression on chromosomes. A trait to be expressed may vary depending on a DNA sequence of a gene, and a genotype may be defined according to the trait to be expressed. Genes at the same location of homologous chromosomes of one chromosome pair may determine one trait, and genotypes of genes present on the respective homologous chromosomes may be different from each other. For example, a human leukocytic antigen (HLA) gene of a person may be present on chromosome 6, a genotype of an X gene of one of homologous chromosomes forming a pair of chromosomes 6 may correspond to a type A, and a genotype of an X gene of the other homologous chromosome may correspond to a type B. Thus, DNA sequences extracted from a gene of one individual may correspond to a pair of DNA sequences having respective genetic traits, and may be expressed as a pair of genetic traits.

SNPs may refer to positions of single nucleotide that differ from individual to individual in a DNA sequence. Due to a difference in DNA sequences appearing in SNPs, different organism individuals belonging to the same species may have different genotypes. For example, referring to FIG. 2, three DNA sequences may correspond to DNA sequences corresponding to the same position among DNA sequences of individuals 210, 220, and 230 belonging to the same species. As shown in FIG. 2, CGTA and TCCGA appear in common in the DNA sequences of the individuals, whereas bases of nucleotides at fifth positions are A 201, G 202, and T 203 that differ from individual to individual. In other words, a position of a fifth nucleotide of FIG. 2 may be called a "SNP". Traits of individuals may be different due to a difference in a part of single nucleotides between DNA sequences.

In an example embodiment, SNP data may include a DNA sequence of at least a portion of a specific gene locus of a specific organism, and information of at least a portion of SNPs included in at least a portion of DNA sequences. A portion of DNA sequences included in SNP data may include single nucleotides that differ from those of DNA sequences of other individuals belonging to the same species. Information of SNPs included in SNP data according to an example embodiment may include position information of single nucleotides that differ from those of DNA sequences of other individuals belonging to the same species among DNA sequences included in the SNP data.

In an example embodiment, a DNA sequence included in SNP data may include a maternal DNA sequence and a paternal DNA sequence. In the following description, a pair of the maternal DNA sequence and the paternal DNA sequence may be referred to as a DNA sequence pair, and a DNA sequence may refer to a maternal DNA sequence and a paternal DNA sequence unless there is a limitation that the DNA sequence refers to only one of a maternal DNA sequence and a paternal DNA sequence.

In an example embodiment, the SNP data 101 to be analyzed may correspond to SNP data extracted from a specific gene of a user to be analyzed. In other words, the SNP data 101 to be analyzed may include at least a portion of a DNA sequence of the specific gene among DNA sequences of the user to be analyzed, and information of at least a portion of SNPs included in the at least portion of the DNA sequence. In an example embodiment, a DNA sequence included in data to be analyzed may include a maternal DNA sequence and a paternal DNA sequence, as described above.

For example, the SNP data 101 to be analyzed may correspond to SNP data of an HLA gene of the user to be analyzed. In this example, the SNP data 101 to be analyzed may include a DNA sequence pair including single nucleotides that are different for each person and that are extracted from an HLA gene present at a specific position of chromosome 6 of a person, and may include position information of single nucleotides that are different for each person.

In the following description, a gene to be analyzed may refer to a specific gene from which the SNP data 101 to be analyzed is extracted. A gene to be analyzed according to an example embodiment may correspond to one of a plurality of genotypes that are predefined according to a DNA sequence of the gene to be analyzed.

According to an example embodiment, the SNP data 101 to be analyzed may be processed to analyze genotypes for each of a maternal DNA sequence and a paternal DNA sequence. In an example embodiment, an operation of processing SNP data to be analyzed may include an operation of separating the SNP data to be analyzed into two pieces of haploid data by phasing the SNP data to be analyzed, and an operation of obtaining two pieces of diploid data by duplicating each of the two pieces of haploid data and pairing the haploid data and duplicated data of the haploid data. In an example embodiment, phasing may refer to an operation of separating a DNA sequence pair into a maternal DNA sequence and a paternal DNA sequence. In an example embodiment, haploid data may refer to SNP data including only one DNA sequence of a maternal DNA sequence and a paternal DNA sequence. In an example embodiment, diploid data may refer to SNP data with a pair of the same DNA sequences generated by duplicating a DNA sequence included in haploid data.

For example, when a paternal DNA sequence "a" and a maternal DNA sequence "b" are included in the SNP data 101 to be analyzed, two pieces of haploid data obtained by phasing SNP data to be analyzed may refer to SNP data with only the paternal DNA sequence "a" and SNP data with only the maternal DNA sequence "b". In this example, two pieces of diploid data in which haploid data and duplicated data of the haploid data are paired may refer to SNP data with a DNA sequence pair including two paternal DNA sequences "a", and SNP data with a DNA sequence pair including two maternal DNA sequences "b".

In an example embodiment, the reference data 102 may include pieces of SNP data with determined genotypes. The SNP data included in the reference data 102 may correspond to the above-described SNP data. For example, when SNP data to be analyzed is extracted from an HLA gene, the SNP data included in the reference data 102 may include at least a portion of a DNA sequence of the HLA gene, and information of at least a portion of SNPs included in the at least portion of the DNA sequence. The at least portion of the DNA sequence may include single nucleotides that differ from those of DNA sequences of other individuals.

In an example embodiment, SNP data with a determined genotype may include at least one SNP data corresponding to one of a plurality of genotypes defined in a gene from which SNP data to be analyzed is extracted. In other words, the SNP data with the determined genotype may correspond to a pair of genotypes corresponding to one of a plurality of genotypes defined in a gene to be analyzed. The SNP data included in the reference data 102 may include a DNA sequence pair formed by two DNA sequences, and each of the DNA sequences may correspond to one of the plurality of genotypes defined in the gene to be analyzed. In other words, a pair of genotypes corresponding to the SNP data included in the reference data 102 may correspond to a pair of genotypes corresponding to each of the DNA sequences of the DNA sequence pair included in the SNP data.

For example, when types A, B, and C are defined as genotypes in a gene from which SNP data to be analyzed is extracted, first SNP data included in the reference data 102 may include a pair of a DNA sequence corresponding to the type A and a DNA sequence corresponding to the type B, and second SNP data included in the reference data 102 may include a pair of a DNA sequence corresponding to the type A and a DNA sequence corresponding to the type C. In this example, a pair of genotypes corresponding to the first SNP data in the reference data 102 may correspond to (type A, type B), and a pair of genotypes corresponding to the second SNP data in the reference data 102 may correspond to (type A, type C).

In an example embodiment, operation 110 of updating the reference data 102 may correspond to an operation of updating the reference data 102 by inserting a marker 103 corresponding to a pair of genotypes of corresponding SNP data into each of a plurality of predetermined regions included in the corresponding SNP data, for each of the plurality of pieces of SNP data included in the reference data 102. Operation 110 of updating the reference data 102 may further include, prior to inserting the marker 103, determining markers 102 corresponding to genotypes of the plurality of pieces of SNP data.

In an example embodiment, the marker 103 may include a marker defined corresponding to each of a plurality of predefined genotypes of a gene to be analyzed. For example, when the plurality of predefined genotypes of the gene to be analyzed correspond to types A, B, and C, the marker 103 may include a first marker defined for the type A, a second marker defined for the type B, and a third marker defined for the type C.

In an example embodiment, the marker 103 may include a binary marker indicating whether a DNA sequence corresponding to a genotype corresponding to a marker is present in SNP data. The binary marker may be marked as "1" indicating that the DNA sequence in the SNP data corresponds to a genotype corresponding to the binary marker, and as "0" indicating that the DNA sequence does not correspond to the genotype corresponding to the binary marker. For example, when the first SNP data corresponds to a pair of genotypes (type A, type B), the first marker defined for the type A may be represented as (1, 0), the second marker defined for the type B may be represented as (0, 1), and the third marker defined for the type C may be represented as (0, 0).

In an example embodiment, the marker 103 may correspond to one DNA sequence included in SNP data, and may be expressed as a tuple of binary markers (for example, a first binary marker, a second binary marker, and a third binary marker) corresponding to genotypes of a gene to be analyzed. For example, when one DNA sequence included in SNP data corresponds to the type A as a genotype, a marker 103 of the DNA sequence may be represented as (1, 0, 0). When the other DNA sequence corresponds to the type B as a genotype, a marker 103 of the other DNA sequence may be represented as (0, 1. 0).

In operation 110 of updating the reference data 102, the plurality of predetermined regions included in the SNP data may refer to a plurality of regions corresponding to a predetermined position and range in a DNA sequence included in the SNP data. In an example embodiment, the plurality of regions may include a plurality of exon regions. An exon may refer to a region in which proteins are synthesized in a DNA sequence of a gene, and a plurality of exons may be present in a DNA sequence of one gene.

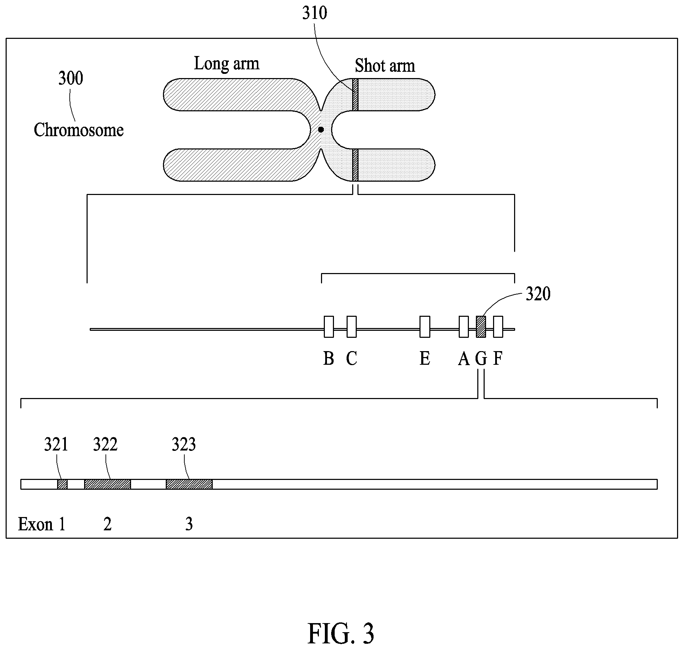

For example, referring to FIG. 3, a gene to be analyzed may correspond to a DNA sequence present at a specific position 310 of a chromosome 300. The gene to be analyzed may be involved in a synthesis of a plurality of proteins, and may be divided into a plurality of sections according to proteins to be synthesized. A DNA sequence corresponding to a section 320 in which a specific protein is synthesized in a DNA sequence of the gene to be analyzed may include a plurality of exons 321, 322 and 323 involved in a synthesis of the specific protein.

In operation 110 of updating the reference data 102, inserting a marker 103 corresponding to a pair of genotypes of the SNP data into the plurality of predetermined regions included in the SNP data may refer to encoding the plurality of predetermined regions with the marker 103. For example, when genotypes of a gene to be analyzed are defined as types A, B, and C, and when first SNP data included in the reference data 102 corresponds to a pair of genotypes (type A, type B), DNA sequences included in the plurality of predetermined regions among DNA sequences included in the first SNP data may be encoded with a binary marker (1, 0) corresponding to the type A, a binary marker (0, 1) corresponding to the type B, and a binary marker (0, 0) corresponding to the type C, respectively.

In an example embodiment, when the plurality of regions of operation 110 correspond to a plurality of exons, a marker 103 corresponding to a pair of genotypes of SNP data may be inserted into a DNA sequence included in each of the exons. For example, referring to FIG. 3, when SNP data included in reference data includes a DNA sequence of FIG. 3, a DNA sequence included in each of exon 1 321, exon 2 322, and exon 3 323 in the DNA sequence may be encoded with a marker 103 corresponding to a pair of genotypes of the SNP data.

Each of pieces of SNP data included in reference data updated by operation 110 according to an example embodiment may include a DNA sequence of a corresponding genotype, information of a SNP included in the DNA sequence, and markers inserted into positions of a plurality of regions in the DNA sequence. The markers inserted in the positions of the plurality of regions in the DNA sequence may correspond to information of markers that encode the plurality of regions in the DNA sequence.

In an example embodiment, a pair of DNA sequences of the SNP data included in the reference data 102 may be separated and used. In other words, in operation 110 of updating the reference data 102, inserting of the marker 103 corresponding to the pair of the genotypes of the SNP data into the plurality of predetermined regions included in the SNP data may refer to inserting a marker 103 corresponding to a genotype of a DNA sequence at a predetermined position in the plurality of predetermined regions for each DNA sequence included in the SNP data. For example, a marker indicating a genotype of one DNA sequence included in reference data may be inserted into a central portion of each of exon regions present in the DNA sequence. A marker indicating a genotype of a DNA sequence according to an example embodiment may correspond to a tuple of binary markers corresponding to a plurality of genotypes.

For example, first SNP data may include a first DNA sequence of a type A, and a second DNA sequence of a type B. In this example, a binary marker indicating the type A may be inserted into a predetermined position (for example, a central position, and the like) in exons included in the first DNA sequence, and a binary marker indicating the type B may be inserted into a predetermined position (for example, a central position, and the like) in exons included in the second DNA sequence. Here, a binary marker indicating a specific genotype may include a tuple including binary markers respectively corresponding to genotypes of a gene to be analyzed. For example, when types A, B, and C are present as genotypes of a gene to be analyzed, a binary marker indicating the type A may correspond to (1, 0, 0), a binary marker indicating the type B may correspond to (0, 1, 0), and a binary marker indicating the type C may correspond to (0, 0, 1).

In an example embodiment, operation 110 of updating the reference data 102 may include an operation of inserting a marker 103 corresponding to a pair of genotypes of SNP data into one of a plurality of predetermined regions included in the SNP data. For example, when the plurality of predetermined regions in the SNP data in the reference data 102 are exons 1 and 2, reference data updated in operation 110 may include SNP data with a marker 103 inserted into the exon 1 only, and SNP data with a marker 103 inserted into the exon 2 only.

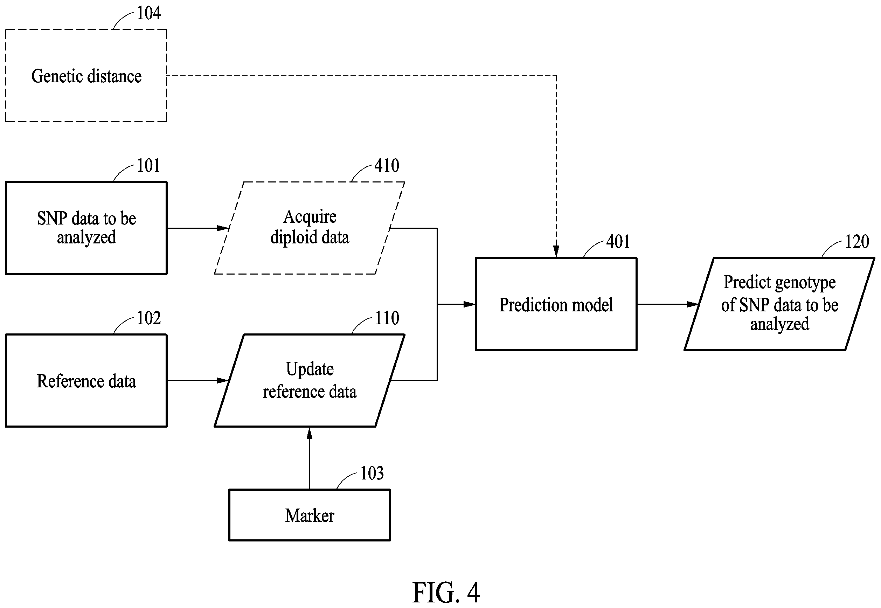

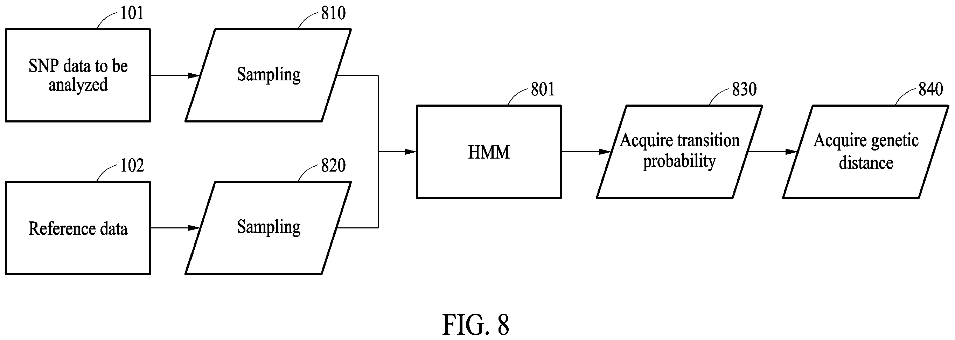

In an example embodiment, operation 120 of predicting a genotype of SNP data to be analyzed may correspond to an operation of predicting the genotype of the SNP data to be analyzed, based on the SNP data 101 to be analyzed and the reference data updated in operation 110. In an example embodiment, operation 120 of predicting the genotype of the SNP data to be analyzed may include an operation of predicting the genotype of the SNP data to be analyzed, based on the SNP data 101 to be analyzed, the reference data updated in operation 110, and a genetic distance 104. Operation 120 of predicting the genotype of the SNP data to be analyzed will be described below with reference to FIGS. 4 to 6B. A method of calculating the genetic distance 104 according to an example embodiment will be described below with reference to FIG. 8.

FIG. 4 illustrates an example of operation 120 of predicting the genotype of the SNP data to be analyzed, using a prediction model.

Referring to FIG. 4, operation 120 of predicting the genotype of the SNP data to be analyzed may include an operation of determining the genotype of the SNP data to be analyzed by inputting the SNP data 101 to be analyzed and the reference data updated in operation 110 to a prediction model 401. The prediction model 401 may correspond to a model that receives the SNP data 101 to be analyzed and the reference data and that outputs a result obtained by calculating probabilities that the SNP data 101 to be analyzed corresponds to a plurality of genotypes predefined in a gene to be analyzed, for each region. Operation 120 of predicting the genotype of the SNP data to be analyzed may include an operation of determining the genotype of the SNP data to be analyzed by inputting the SNP data 101 to be analyzed, the reference data updated in operation 110, and the genetic distance 104 to the prediction model 401.

In an example embodiment, the prediction model 401 may include a BEAGLE model and an artificial neural network model. In the following description, an example in which the prediction model 401 is a BEAGLE model will be described, however, there is no limitation thereto.

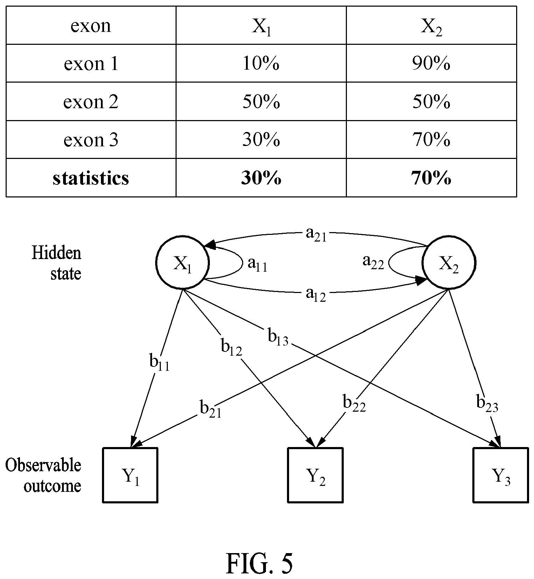

In an example, the prediction model 401 may include a model that predicts a genotype from SNP data based on a hidden Markov model (HMM). In this example, the prediction model 401 may include hidden states corresponding to a plurality of genotypes included in a gene to be analyzed, observable data corresponding to SNP data, transition probabilities between the hidden states, and emission probabilities from each of the hidden states to observable data. For example, referring to FIGS. 5 to 6B, the prediction model 401 may include hidden states corresponding to a plurality of genotypes $X_1$ and $X_2$ predefined in a gene to be analyzed, observable data corresponding to DNA sequences $Y_1$, $Y_2$, and $Y_3$ included in SNP data to be analyzed, transition probabilities an, $a_{12}$, $a_{21}$, and $a_{22}$ between states, and emission probabilities $b_{11}$, $b_{12}$, $b_{13}$, $b_{21}$, $b_{22}$, and $b_{23}$ from each of the states to each observable data.

Referring to FIG. 4, operation 120 of predicting the genotype of the SNP data to be analyzed may include an operation of calculating probabilities that the SNP data to be analyzed corresponds to genotypes of a plurality of pieces of SNP data for each region by inputting the SNP data 101 to be analyzed and the updated reference data to the prediction model 401, and an operation of determining the genotype of the SNP data 101 to be analyzed, based on the probabilities. More specifically, the prediction model 401 may calculate probabilities that the SNP data 101 to be analyzed corresponds to each of the genotypes, for each of the plurality of regions with the inserted marker 103 in the reference data updated in operation 110, and a probability that the SNP data 101 to be analyzed corresponds to one genotype may be calculated as an average of probabilities of corresponding to the genotype calculated for each of the plurality of regions. In this example, by comparing average probabilities calculated for each of the plurality of regions, a genotype with a highest average probability may be predicted as the genotype of the SNP data to be analyzed.

For example, referring to FIG. 5, in operation 110, for each SNP data included in the reference data, the reference data may be updated by inserting a marker into each of a plurality of regions, for example, exons 1, 2, and 3, included in corresponding SNP data. In this example, the prediction model 401 may calculate probabilities of corresponding to genotypes $X_1$, and $X_2$, for each of the plurality of regions, for example, the exons 1, 2, and 3. In operation 120, probabilities of corresponding to each of genotypes may be represented as an average of probabilities calculated for each of the plurality of regions. For example, in FIG. 5, a probability of corresponding to the genotype $X_1$ may be expressed as 30% by averaging 10% calculated for the exon 1, 50% calculated for the exon 2, and 30% calculated for the exon 3, and a probability of corresponding to the genotype $X_2$ may be expressed as 70% by averaging 90% calculated for the exon 1, 50% calculated for the exon 2, and 70% calculated for the exon 3. In this example, the genotype of the SNP data to be analyzed may be determined as the genotype $X_2$ with a higher average probability than the genotype $X_1$.

In an example embodiment, operation 120 may include an operation of setting a plurality of parameters indicating lengths of DNA sequences for analyzing the SNP data to be analyzed, based on a plurality of pieces of SNP data included in the updated reference data, an operation of calculating probabilities that the SNP data to be analyzed corresponds to genotypes of the plurality of pieces of SNP data for each combination of the regions and the parameters, by inputting the parameters, the SNP data to be analyzed and the updated reference data to a prediction model, and an operation of determining the genotype of the SNP data to be analyzed, based on the probabilities for each combination of the regions and the parameters.

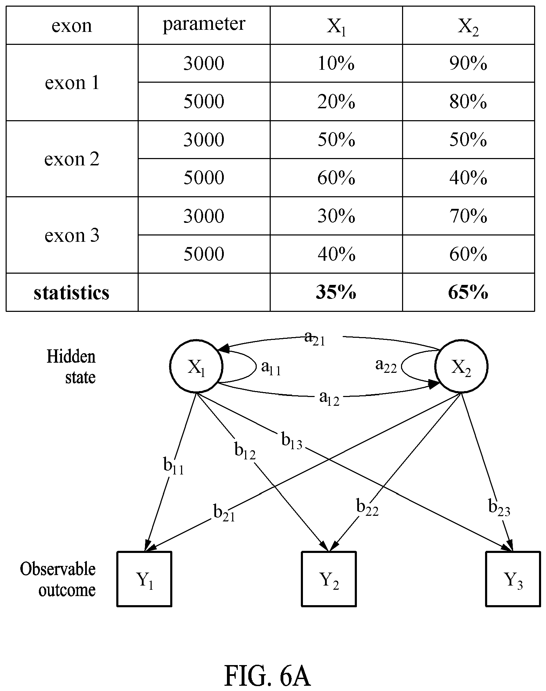

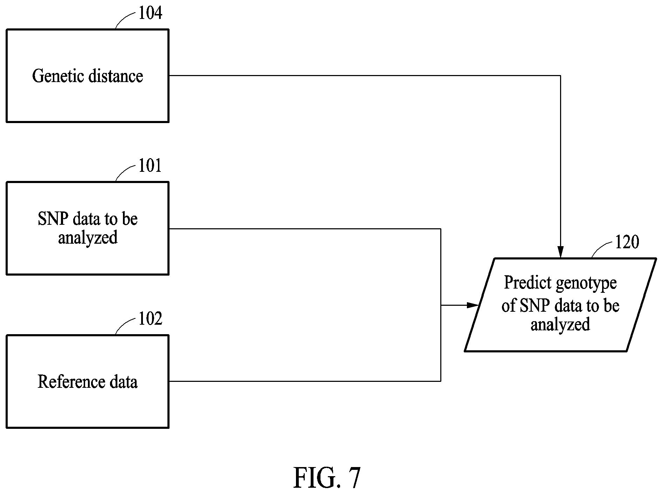

For example, referring to FIG. 6A, in operation 110, for each of SNP data included in the reference data, a marker may be inserted into each of a plurality of regions, for example, exons 1, 2, and 3, included in corresponding SNP data, and the reference data may be updated. Also, a plurality of parameters indicating lengths of DNA sequences for analyzing the SNP data to be analyzed may be set to 3000 and 5000. In this example, the prediction model 401 may calculate probabilities of corresponding to genotypes $X_1$, and $X_2$ for each combination of the plurality of regions, for example, the exons 1, 2, and 3, and the parameters 3000 and 5000. For example, in FIG. 6A, the probabilities of corresponding to the genotypes $X_1$, and $X_2$ for each combination of the plurality of regions, for example, the exons 1, 2, and 3, and the parameters 3000 and 5000 may include 10% calculated for the exon 1 by setting a parameter as 3000, and 20% calculated for the exon 1 by setting a parameter as 5000. Referring to FIG. 7, a probability of corresponding to the genotype $X_1$ may be 35% that is an average of probabilities calculated for each combination of the plurality of regions, for example, the exons 1, 2, and 3, and the parameters 3000 and 5000, and a probability of corresponding to the genotype $X_2$ may be 65% that is an average of probabilities calculated for each combination of the plurality of regions, for example, the exons 1, 2, and 3, and the parameters 3000 and 5000. In this example, the genotype of the SNP data to be analyzed may be determined as the genotype $X_2$ with a higher average probability than the genotype $X_1$.

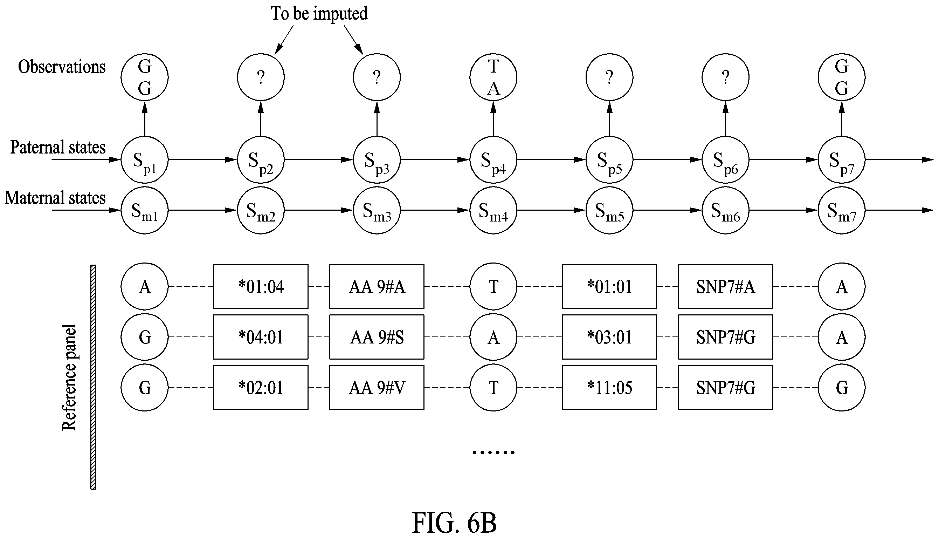

Although an example of an HMM with a general circulation structure has been described above with reference to FIGS. 5 and 6A for convenience of description, the HMM may have a structure of FIG. 6B. Referring to FIG. 6B, a state may transition from left to right by a genomic position.

In an example embodiment, referring to FIG. 4, the SNP data 101 to be analyzed may be processed through operation 410 of acquiring two pieces of diploid data and may be input to the prediction model 401. The SNP data 101 to be analyzed may be processed through operation 410 of acquiring the diploid data. As described above, the SNP data 101 to be analyzed may include a DNA sequence pair including a maternal DNA sequence and a paternal DNA sequence, and an operation of processing SNP data to predict a genotype of each DNA sequence may be included. In an example embodiment, an operation of processing SNP data may include separating SNP data into two pieces of haploid data by phasing the SNP data. In other words, an operation of separating SNP data to be analyzed into two pieces of haploid data by phasing the SNP data may include separating the SNP data to be analyzed into maternal SNP data and paternal SNP data. In an example embodiment, the maternal SNP data may correspond to SNP data with a DNA sequence inherited from the mother, and the paternal SNP data may correspond to SNP data with a DNA sequence inherited from the father. An operation of processing SNP data to be analyzed according to an example embodiment may include separating the SNP data to be analyzed into two pieces of haploid data by phasing the SNP data to be analyzed, and obtaining two pieces of diploid data by duplicating each of the two pieces of haploid data and pairing the haploid data and duplicated data of the haploid data. According to an example embodiment, the SNP data 101 to be analyzed may be input to a prediction model, without passing through an operation of phasing and processing.

In an example embodiment, operation 120 of predicting the genotype of the SNP data to be analyzed may include predicting the genotype of the SNP data to be analyzed, based on each of maternal diploid data and paternal diploid data acquired in operation 410, and the updated reference data. In an example embodiment, operation 120 of predicting the genotype of the SNP data to be analyzed may include predicting a genotype of corresponding diploid data by inputting the corresponding diploid data and the updated reference data to the prediction model 401, for each of the two pieces of diploid data acquired in operation 410.

In an example embodiment, operation 120 of predicting the genotype of the SNP data to be analyzed may include calculating a genetic distance 104 between a plurality of markers 103 corresponding to the genotypes of the plurality of pieces of SNP data, and predicting the genotype of the SNP data to be analyzed, based on the SNP data 101 to be analyzed, the reference data updated in operation 110, and the genetic distance. A method of calculating a genetic distance according to an example embodiment will be further described below with reference to FIG. 8.

FIG. 7 illustrates a method of predicting a genotype of SNP data to be analyzed, based on the SNP data to be analyzed, reference data, and a genetic distance according to an example embodiment.

Referring to FIG. 7, a method of predicting a genotype using SNP data according to an example embodiment may include acquiring SNP data 101 to be analyzed, acquiring reference data 102 including a plurality of pieces of SNP data with determined genotypes, acquiring a genetic distance 104 between states, and operation 120 of predicting a genotype of SNP data to be analyzed based on the genetic distance 104, the reference data 102, and the SNP data 101 to be analyzed.

Although not shown in FIG. 7, operation 120 of predicting the genotype of the SNP data to be analyzed may include predicting, using the prediction model 401, the genotype of the SNP data to be analyzed. In an example embodiment, the prediction model 401 may receive, as inputs, a genetic distance 104 between DNA sequences corresponding to genotypes, in addition to the SNP data to be analyzed and updated reference data.

In an example embodiment, the genetic distance 104 may be obtained through an operation of sampling the SNP data 101 to be analyzed and the plurality of pieces of SNP data included in the reference data 102, an operation of calculating a transition probability between states corresponding to genotypes of the plurality of pieces of SNP data in an HMM based on the sampled data, and acquiring a genetic distance by converting the transition probability between the states. A method of acquiring a genetic distance according to an example embodiment will be further described below with reference to FIG. 8.

FIG. 8 is a diagram illustrating a method of acquiring a genetic distance according to an example embodiment.

According to an example embodiment, a genetic distance may correspond to a parameter indicating a difference between two DNA sequences determined as different genotypes. For example, when a DNA sequence determined as a type A and a DNA sequence determined as a type B are similar, a genetic distance may have a relatively low value. When the DNA sequences are different, the genetic distance may have a relatively high value. The genetic distance according to an example embodiment may include, for example, a genetic distance that is measured from public data and that is known, or a genetic distance acquired based on an operation of calculating a genetic distance according to an example embodiment.

Referring to FIG. 8, an operation of calculating a genetic distance according to an example embodiment may include operations 810 and 820 of sampling SNP data 101 to be analyzed and reference data 102 including a plurality of pieces of SNP data, operation 830 of calculating a transition probability between states corresponding to genotypes of the plurality of pieces of SNP data included in the reference data 102 in an HMM 801 based on the sampled data, and operation 840 of acquiring a genetic distance between the states by converting the transition probability between the states.

In an example embodiment, operation 820 of sampling the reference data 102 may correspond to an operation of extracting at least a portion of the pieces of SNP data from the reference data 102. Operation 810 of sampling the SNP data 101 to be analyzed may correspond to an operation of extracting at least a portion of a plurality of pieces of SNP data when the plurality of pieces of SNP data are included in the SNP data to be analyzed. The sampled SNP data and the sampled reference data may be input to the HMM 801 used to calculate a transition probability. Operation 830 of calculating the transition probability may include calculating a transition probability between states corresponding to genotypes of the plurality of pieces of SNP data in the HMM 801, using an algorithm that measures a transition probability. The algorithm that measures the transition probability may include, for example, a Baum-Welch algorithm. Operation 840 of acquiring the genetic distance between the states by converting the transition probability between the states may correspond to an operation of converting a transition probability between states to a genetic distance between states using the following equation:

[Equation 1]

$$\tau = 1 - e^{-4Nr/H}$$

In Equation 1, $\tau$ denotes the transition probability between the states calculated in operation 830, r denotes a genetic distance, N denotes an effective population size of a target race to be analyzed (effective population sizes for each race are known. For example, an effective population size of westerners may be set to "10,000"), and H corresponds to a number of states of an HMM. Each of pieces of SNP data included in the sampled reference data according to an example embodiment may correspond to SNP data extracted from a single organism, and accordingly H may correspond to a number of organism individuals from which SNP data included in the sampled reference data is extracted.

In an example embodiment, the genetic distance 104 may include a genetic distance between markers defined corresponding to each of a plurality of predefined genotypes of a gene to be analyzed. In this example, the genetic distance between the markers may include a genetic distance between DNA sequences determined as a genotype corresponding to each of the markers.

According to an example embodiment, a genotype of SNP data to be analyzed may be predicted based on a genetic distance between genotypes acquired in operation 840, thereby increasing an accuracy of prediction of a genotype.

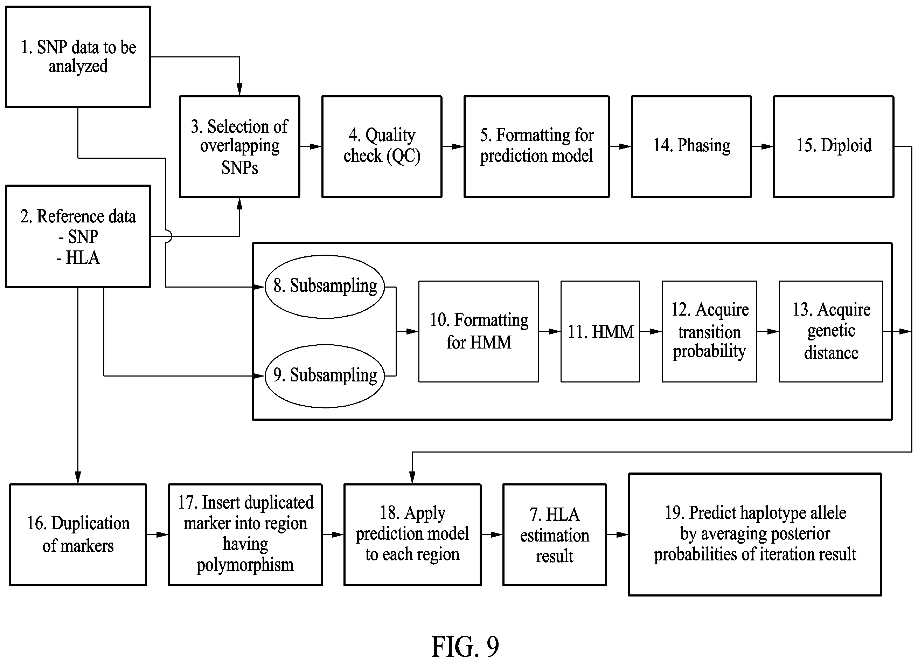

FIG. 9 illustrates an overall process of a method of predicting a genotype using SNP data according to an example embodiment.

[Idea 1]

According to an example embodiment, when a prediction model is running by fusing (binary) markers with SNP data included in reference data, a genetic distance between the markers may be given as an input value. When the genetic distance is provided as an input value, a method of using a genetic distance that is measured from public data such as HapMap and that is known may exist.

However, according to example embodiments, a method of inferring an accurate genetic distance using data (for example, SNP data to be analyzed and reference data) used by a current user and of using the inferred genetic distance in a prediction algorithm may be included. Thus, an accuracy of prediction of a genotype of SNP data to be analyzed may be enhanced.

A scheme of calculating a genetic distance according to an example embodiment is described below.

1) Subsampling for extracting a portion of pieces of SNP data from SNP data to be analyzed and reference data is performed.

2) Using a MaCH 1.0 algorithm, a transition probability is measured using a Baum-Welch algorithm in an HMM in which the SNP data to be analyzed is formed in a mosaic pattern of pieces of SNP data included in the sampled reference data.

3) The measured transition probability is converted to a genetic distance using the following equation:

$$\tau = 1 - e^{-4Nr/H}$$

τ: Transition probability
N: Effective population size
r: Genetic distance
H: Number of organisms included in the sampled reference data (= number of states of the HMM)

4) The genetic distance is used as an input value in a prediction algorithm (for example, Beagle v4).

[Effect of Idea 1]

An effect of increasing a prediction accuracy may be obtained with the idea 1. In test data (for example, HapMap European (N=124), and reference data of "5,000" people was used, based on a high resolution (4-digit) average accuracy), an existing model (SNP2HLA) shows an accuracy of 95.0%, whereas the accuracy was increased to 97.6% and errors were reduced when the idea 1 is used.

[Idea 2]

When (binary) markers are fused with SNP data, there was an issue of a position into which the markers need to be inserted, and in the existing model, a scheme of inserting the markers into a central portion of a gene was selected simply.

However, since the fact that close markers have a kind of correlation called linkage disequilibrium is utilized in genotype prediction algorithms, the correlation decreases as a distance between markers increases. Thus, an accuracy of a prediction result may decrease. It is known that exons 2, 3 and 4 are most polymorphic in HLA class 1 and that exons 2 and 3 are most polymorphic in HLA class 2, and variation information in the above exons decisively contributes to determining a genotype of a gene.

Accordingly, placing (binary) markers in the above polymorphic positions may be most effective when considering the linkage disequilibrium. However, if markers are positioned on an exon 2, the markers may be too far from an exon 4, and if the markers are positioned on the exon 4, the markers may be too far from the exon 2, which may cause a problem.

In idea 2 according to example embodiments, the markers may be duplicated and each of the duplicated markers may be inserted into a central portion of each exon. Here, there is still a problem of how to finally predict a genotype using the markers that are duplicated and inserted. To this end, a scheme of phasing SNP data to be analyzed may be used first.

Phasing may refer to a process of distinguishing between chromosomes inherited from the mother and chromosomes inherited from the father. If phasing is completed in computation based on SNP data, a single piece of SNP data to be analyzed may be divided into two pieces of haploid data. Generally, a prediction model (for example, Beagle v4) may not receive haploid data in an input format, and accordingly homozygous diploid data may be generated by copying and doubling each of the haploid data. In other words, through the phasing, a DNA sequence of a single individual may be divided as if it is DNA sequences of two persons.

Subsequently, after running a prediction model using each diploid data (in fact, haploid data in information), posterior probabilities obtained from regions into which duplicated markers are inserted may be averaged to determine a genotype with a highest posterior probability.

For example, posterior probabilities may be obtained from markers inserted into exons 2, 3 and 4. In this example, to average the posterior probabilities, only averaging of information about genotypes in the same haplotype may be meaningful.

The present scheme is performed as follows.

1) Markers are duplicated and located in central portions of exons 2, 3 and 4 (or exons 2 and 3 in the case of class 2) during updating of reference data.

2) Pieces of haploid data are generated by phasing SNP data to be analyzed.

3) Pieces of diploid data are generated by copying and pasting the haploid data.

4) A prediction model is run using updated reference data based on the diploid data of the SNP data to be analyzed.

5) Posterior probabilities for each genotype are calculated from the markers located in each exon in a result.

6) The posterior probabilities are averaged for multiple exons.

7) A genotype with a highest average posterior probability is predicted as a genotype of the haploid data.

[Effect of Idea 2]

An effect of increasing a prediction accuracy may also be obtained with the idea 2. In the same test data, when only the idea 2 is used, the accuracy of 97.5% may be obtained similarly to when the idea 1 is used. When the ideas 1 and 2 are simultaneously applied, the accuracy of 98.0% is obtained, which indicates that the accuracy is further increased and an error rate is also reduced in comparison to when the ideas 1 and 2 are individually applied.

To apply the idea 2, a genotype with a highest posterior probability may be predicted by performing phasing. In the above process, since only one genotype is predicted for each haploid, an error in predicting a plurality of genotypes due to a collision between markers may be eliminated.

[Idea 3]

By updating a prediction model (for example, Beagle v3) that was internally used to, for example, Beagle v4 or v5, time and memory were reduced.

[Effect of Idea 3]

Using a recently developed prediction model, an effect of reducing time and memory by several times or more may be obtained.

The example embodiments described herein may be implemented using hardware components, software components, and/or a combination thereof. For example, a processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such as parallel processors.

Software may include a computer program, a piece of code, an instruction, or some combination thereof, for independently or collectively instructing or configuring the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. In particular, the software and data may be stored by one or more non-transitory computer readable recording mediums.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs or DVDs; magneto-optical media such as floptical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

**Claims**

1. A method of predicting a genotype using single nucleotide polymorphism (SNP) data, the method comprising:

   acquiring SNP data to be analyzed;
   acquiring reference data comprising a plurality of pieces of SNP data with determined genotypes;
   updating the reference data by inserting a marker corresponding to a genotype of corresponding SNP data into each of a plurality of predetermined regions included in the corresponding SNP data, for each of the plurality of pieces of SNP data included in the reference data; and
   predicting a genotype of the SNP data to be analyzed, based on the SNP data to be analyzed and the updated reference data.

2. The method of claim 1, wherein the updating of the reference data comprises inserting a binary marker corresponding to the genotype of the corresponding SNP data into a plurality of exons included in the corresponding SNP data, for each of the plurality of pieces of SNP data included in the reference data.

3. The method of claim 1, wherein the predicting of the genotype of the SNP data to be analyzed comprises:

   calculating probabilities that the SNP data to be analyzed corresponds to the genotypes of the plurality of pieces of SNP data for each region, by inputting the SNP data to be analyzed and the updated reference data to a prediction model; and
   predicting the genotype of the SNP data to be analyzed, based on the probabilities.

4. The method of claim 1, wherein the predicting of the genotype of the SNP data to be analyzed comprises:

   setting a plurality of parameters indicating lengths of nucleic acid sequences for analyzing the SNP data to be analyzed, based on the plurality of pieces of SNP data included in the updated reference data;
   calculating probabilities that the SNP data to be analyzed corresponds to the genotypes of the plurality of pieces of SNP data for each combination of the regions and the parameters, by inputting the parameters, the SNP data to be analyzed and the updated reference data to a prediction model; and
predicting the genotype of the SNP data to be analyzed, based on the probabilities.

5. The method of claim 1, wherein the predicting of the genotype of the SNP data to be analyzed comprises:

   calculating a genetic distance between a plurality of markers corresponding to the genotypes of the plurality of pieces of SNP data; and
   predicting the genotype of the SNP data to be analyzed, based on the genetic distance, the SNP data to be analyzed, and the updated reference data.

6. The method of claim 5, wherein the calculating of the genetic distance comprises:

   sampling the SNP data to be analyzed and the plurality of pieces of SNP data;
   calculating a transition probability between states corresponding to the genotypes of the plurality of pieces of SNP data in a hidden Markov model (HMM), based on the sampled data; and
   acquiring a genetic distance between the states by converting the transition probability between the states.

7. The method of claim 1, further comprising:

   separating the SNP data to be analyzed into two pieces of haploid data by phasing the SNP data to be analyzed; and
   obtaining two pieces of diploid data by duplicating each of the two pieces of haploid data and pairing the haploid data and duplicated data of the haploid data.

8. The method of claim 7, wherein the predicting of the genotype of the SNP data to be analyzed comprises predicting a genotype of corresponding diploid data by inputting the corresponding diploid data and the updated reference data to a prediction model, for each of the two pieces of diploid data.

9. The method of claim 7, wherein the separating of the SNP data to be analyzed into the two pieces of haploid data by phasing the SNP data to be analyzed comprises separating the SNP data to be analyzed into maternal SNP data and paternal SNP data.

10. The method of claim 1, further comprising:
    determining markers corresponding to the genotypes of the plurality of pieces of SNP data.

11. The method of claim 1, wherein the SNP data to be analyzed comprises:

at least a portion of a DNA sequence of a user to be analyzed; and

information of at least a portion of SNPs included in the at least portion of the DNA sequence.

12. The method of claim 1, wherein the reference data comprises at least one SNP data corresponding to one of a plurality of genotypes defined in a gene from which the SNP data to be analyzed is extracted.

13. The method of claim 1, wherein each of the plurality of pieces of SNP data included in the updated reference data comprises:

a DNA sequence of a corresponding genotype;

information of a SNP included in the DNA sequence; and

markers inserted into positions of the regions in the DNA sequence.

14. The method of claim 1, wherein
the SNP data to be analyzed comprises SNP data extracted from a human leukocytic antigen (HLA) gene, and
the genotypes comprise a plurality of genotypes defined in the HLA gene.

15. A method of predicting a genotype using single nucleotide polymorphism (SNP) data, the method comprising:

acquiring SNP data to be analyzed;

acquiring reference data comprising a plurality of pieces of SNP data with determined genotypes;

sampling the SNP data to be analyzed and the plurality of pieces of SNP data;

calculating a transition probability between states corresponding to the genotypes of the plurality of pieces of SNP data in a hidden Markov model (HMM), based on the sampled data;

acquiring a genetic distance between the states by converting the transition probability between the states; and

predicting a genotype of the SNP data to be analyzed, based on the genetic distance, the reference data, and the SNP data to be analyzed.

16. A computer program stored in a medium to execute the method of one of claims 1 to 15 in combination with hardware.

17. An apparatus for predicting a genotype using single nucleotide polymorphism (SNP) data, the apparatus comprising:

a memory configured to store SNP data to be analyzed, and reference data comprising a plurality of pieces of SNP data with determined genotypes; and

a processor configured to update the reference data by inserting a marker corresponding to a genotype of corresponding SNP data into each of a plurality of predetermined regions included in the corresponding SNP data, for each of the plurality of pieces of SNP data included in the reference data, and to predict a genotype of the SNP data to be analyzed, based on the SNP data to be analyzed and the updated reference data.

18. The apparatus of claim 17, wherein, to update the reference data, the processor is configured to insert a binary marker corresponding to the genotype of the corresponding SNP data into a plurality of exons included in the corresponding SNP data, for each of the plurality of pieces of SNP data included in the reference data.

19. The apparatus of claim 17, wherein, to predict the genotype of the SNP data to be analyzed, the processor is configured to calculate a genetic distance between a plurality of markers corresponding to the genotypes of the plurality of pieces of SNP data and to predict the genotype of the SNP data to be analyzed, based on the genetic distance, the SNP data to be analyzed, and the updated reference data.

20. The apparatus of claim 17, wherein
the SNP data to be analyzed comprises SNP data extracted from a human leukocytic antigen (HLA) gene, and
the genotypes comprise a plurality of genotypes defined in the HLA gene.

FIG. 1

FIG. 2

FIG. 3

FIG. 4

| exon | $X_1$ | $X_2$ |
|---|---|---|
| exon 1 | 10% | 90% |
| exon 2 | 50% | 50% |
| exon 3 | 30% | 70% |
| **statistics** | **30%** | **70%** |

FIG. 5

| exon | parameter | $X_1$ | $X_2$ |
|---|---|---|---|
| exon 1 | 3000 | 10% | 90% |
|  | 5000 | 20% | 80% |
| exon 2 | 3000 | 50% | 50% |
|  | 5000 | 60% | 40% |
| exon 3 | 3000 | 30% | 70% |
|  | 5000 | 40% | 60% |
| **statistics** |  | **35%** | **65%** |

FIG. 6A

FIG. 6B

104

Genetic distance

101

SNP data to be
analyzed

102

Reference data

120

Predict genotype
of SNP data to be
analyzed

FIG. 7

```
  ┌─────────────┐ 101        ╱────────────╱ 810
  │ SNP data to │           ╱  Sampling  ╱
  │ be analyzed │          ╱────────────╱
  └─────────────┘                          ┐
                                           │      ┌──────┐ 801      ╱────────────╱ 830     ╱────────────╱ 840
                                           ├────▶ │ HMM  │ ──────▶ ╱  Acquire   ╱ ──────▶ ╱  Acquire   ╱
  ┌─────────────┐ 102        ╱────────────╱ 820   └──────┘        ╱ transition ╱          ╱  genetic   ╱
  │ Reference   │           ╱  Sampling  ╱   ┘                   ╱ probability╱          ╱  distance  ╱
  │ data        │          ╱────────────╱                      ╱────────────╱          ╱────────────╱
  └─────────────┘
```

FIG. 8

FIG. 9

EP 3 843 101 A1

## INTERNATIONAL SEARCH REPORT

| International application No. |
|---|
| **PCT/KR2020/000436** |

**A.    CLASSIFICATION OF SUBJECT MATTER**

*G16B 20/20(2019.01)i, G16B 30/00(2019.01)i, G16B 40/00(2019.01)i, G16B 50/00(2019.01)i*

According to International Patent Classification (IPC) or to both national classification and IPC

**B.    FIELDS SEARCHED**

Minimum documentation searched (classification system followed by classification symbols)
G16B 20/20; G16B 30/00; G16B 40/00; G16B 50/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: genotype prediction, imputation, SNP data, HLA allele, marker insertion, exon

**C.    DOCUMENTS CONSIDERED TO BE RELEVANT**

| Category* | Citation of document, with indication, where appropriate, of the relevant passages | Relevant to claim No. |
|---|---|---|
| X | COOK, S et al. PgmNr 1557: CookHLA: Accurate HLA imputation. American Society of Human Genetics 2018 Annual Meeting. October 2018, pages 1-2 See the entire document. | 1-3,5,7-14,16-20 |
| A | | 4,6,15 |
| X | COOK, Seungho et al. CookHLA(v0.3): Accurate, efficient, and memory-efficient HLA imputation. 14회 KOGO 동계심포지엄(The 14th KOGO Winter Symposium). February 2018, page 178, I-05 See the entire document. | 1-3,5,7-14,16-20 |
| X | COOK, Seungho et al. CookHLA: Accurate HLA imputation from genomic data. 27회 국제 KOGO 정기학술대회(The 27th International KOGO Annual Conference). September 2018, page 165, I-11 See the entire document. | 1-3,5,7-14,16-20 |
| X | PILLAI, Nisha Esakimuthu et al. Predicting HLA alleles from high-resolution SNP data in three Southeast Asian populations. Human Molecular Genetics. 2014, vol. 23, no. 16, pages 4443-4451 See materials and methods section; and figure 2. | 1,5,10,16-17,19 |
| X | COOK, Seungho et al. MergeReference: a tool for merging reference panels for HLA imputation. Genomics & Informatics. 2017, vol. 15, no. 3, pages 108-111 See methods section. | 1,16-17 |

| ☐ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 APRIL 2020 (22.04.2020) | **22 APRIL 2020 (22.04.2020)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)